# EUROPÄISCHE PATENTANMELDUNG

(11) **EP 4 260 885 A1**
(43) Veröffentlichungstag der Anmeldung: **18.10.2023**
(21) Anmeldenummer: 22167701.6
(22) Anmeldetag: 11.04.2022
(51) Int. Cl.: A61M 5/142, A61M 5/168, A61M 5/172, G16H 20/17, A61M 39/28

(54) **INFUSIONSPUMPE SOWIE VERFAHREN ZU DEREN BETRIEB**

(71) Anmelder: Conncons GmbH, 01445 Radebeul (DE)
(72) Erfinder: Gommel, Christoph, 01445 Radebeul (DE); Hampe, Jochen, 01277 Dresden (DE); Schurig, Carsten, 01445 Radebeul (DE)
(74) Vertreter: Witte, Weller und Partner Patentanwälte mbB Stuttgart

(57) **Zusammenfassung**

Bekannt sind Infusionspumpen (10) zur Versorgung eines Patienten (2) mit mindestens einem Medikament. Es wird eine Infusionspumpe (10) vorgeschlagen, die über einen elektrisch betriebenen Pumpaktor (24) und vorzugsweise mehrere Ventilaktoren (22A) verfügt. Die Infusionspumpe (10) weist an der Basiseinheit (20) ein Steuergerät (30) zur Steuerung mindestens des Pumpaktors (24) und der Ventilaktoren (22A) auf, wobei das Steuergerät (30) mit mindestens einer ersten Datenschnittstelle (32A) zur Verbindung mit dem mindestens einen Medikamentenspeicher (110) und mit einer zweiten Datenschnittstelle (32B) zur Verbindung mit dem Patienten verbunden ist. Das Steuergerät (30) ist dafür ausgebildet, patientenbezogene und medikamentenbezogene Daten über die Datenschnittstellen (32A, 32B) zu erfassen und mindestens den Pumpaktor (24) in Abhängigkeit der über die Datenschnittstellen (32A, 32B) erfassten Daten zu steuern.

## Beschreibung

### ANWENDUNGSGEBIET UND STAND DER TECHNIK

Die Erfindung betrifft Infusionspumpe zur Versorgung eines Patienten Medikamenten in flüssiger Form.

Übliche Infusionspumpen verfügen über eine Pumpeinrichtung in Art einer Peristaltikpumpe. An einem Eingang der Pumpeinrichtung werden Medikamentenspeicher angeschlossen. Diese sind jeweils mit einem manuell zu öffnenden und zu schließenden Ventil versehen. Die Flüssigkeit aus den Medikamentenspeichern wird im Rahmen der Infusionsbehandlung nacheinander dem Patienten über eine Zugangseinrichtung wie einen Venenkatether zugeführt.

Die Infusionsbehandlung läuft üblicherweise unter Überwachung und mit manuellen Eingriffen zum Wechsel der Ventilstellung der Medikamentenspeicher ab.

Die Konfiguration einer Infusionsanordnung umfassend die Infusionspumpe, der daran ausgangsseitig angeschlossene patientenseitigen Zugangseinrichtung sowie der an der Infusionspumpe eingangsseitig angeschlossenen Medikamentenspeicher sowie die manuelle Handhabung der Infusionsbehandlung und die damit einhergehende Überwachung und Dokumentation der Infusionsbe-\ handlung stellen einen erheblichen Aufwand dar. Darüber hinaus ist die Gefahr von Fehlern gegeben. Es muss genau und aufwändig geprüft werden, dass die angeschlossenen Medikamente die richtigen sind und die jeweiligen Infusionsparameter der einzelnen Medikamente gemäß einem Therapieplan richtig vorgegeben und eingehalten werden.

### AUFGABE UND LÖSUNG

Aufgabe der Erfindung ist es, eine Infusionspumpe und ein korrespondierendes Betriebsverfahren zur Verfügung zu stellen, die den Personalaufwand und/oder die Gefahr eines Behandlungsfehlers im Rahmen der Infusionsbehandlung senkt.

Hierfür wird erfindungsgemäß primär eine Infusionspumpe zur Versorgung eines Patienten mit Medikamenten in flüssiger Form vorgeschlagen, die in besonderer Form zur Datenkommunikation mit patientenspezifischen und medikamentenspezifischen Gegenstellen ausgebildet ist.

Eine erfindungsgemäße Infusionspumpe weist in an sich bekannter Weise eine Basiseinheit und eine hieran anzukoppelnde Leitungseinheit auf.

Die Leitungseinheit umfasst alle Fluidleitungen und fluidführenden Flächen, wobei diese zumindest zum Teil formflexibel ausgestaltet sind. Die Leitungseinheit weist mindestens einen Fluideingang zum Anschluss mindestens eines Medikamentenspeichers sowie mindestens einen Ausgang zum Anschluss des Patienten auf. Die Zahl der Fluideingänge beträgt vorzugsweise zwei oder mehr, insbesondere fünf oder mehr. Hierüber lassen sich mehrere Medikamentenspeicher sowie ggf. zusätzlich ein Speicher für Spülflüssigkeit anschließen.

Die Fluidausgänge und Fluideingänge sind jeweils in Art eines Anschlussstücks ausgebildet, insbesondere in Form eines Luer-Konnektors gemäß der Normenfamilie ISO 80369. Sie dienen dem Anschluss an ein entsprechendes Gegenanschlussstück einer Schlauchleitung.

An der Basiseinheitder Infusionspumpe ist mindestens ein elektrisch betriebener Pumpaktor vorgesehen, der von außen auf die Leitungseinheit wirkt und damit eine Peristaltikpumpe bildet. Vorzugsweise handelt es sich um eine Schieberperistaltikpumpe, die einen besonders gleichmäßig Fluss gewährleistet. Der Pumpaktor bewirkt, dass Flüssigkeit von einem Fluideingang und damit aus dem Medikamentenspeicher zum Fluidausgang und damit zum Patienten gefördert wird.

Neben dem Pumpaktor weist die Basiseinheit vorzugsweise mindestens einen elektrisch steuerbaren Ventilaktor als Teil einer Ventileinrichtung auf, mittels derer jeweils ein Fluideingang getrennt werden kann, so dass Flüssigkeit von diesem Fluideingang nicht mehr zur Pumpeinrichtung und dem Fluidausgang strömen kann. Vorzugsweise ist allen Fluideingängen jeweils ein Ventilaktor zugeordnet, um selektiv stets nur von einem Fluideingang einen Zufluss zur Pumpeinrichtung zu gestatten. Auch dem Fluidausgang kann eine Ventileinrichtung zugeordnet sein.

Die Basiseinheit ist weiterhin auch Träger eines Steuergerätes. Dieses Steuergerät steuert zu mindest den Pumpaktor und vorzugsweise weiterhin auch die Ventilaktoren. Das Steuergerät verfügt über Datenschnittstellen, nämlich eine erste Datenschnittstelle mittels derer das Steuergerät mit dem mindestens einen Medikamentenspeicher für den Datenaustausch gekoppelt ist, und eine zweite Datenschnittstelle mittels derer das Steuergerät mit dem Patienten bzw. einer dem Patienten unmittelbar zugeordneten Gegenstelle für den Datenaustausch gekoppelt ist.

Das Steuergerät ist dafür ausgebildet, patientenbezogene und medikamentenbezogene Daten über die Datenschnittstellen zu erfassen. Diese Daten werden vom Steuergerät beim Steuern des Pumpaktors sowie vorzugsweise der Ventilaktoren herangezogen.

Eine erfindungsgemäße Infusionspumpe führt dadurch zu großen Vorteilen in der Handhabung. Dadurch, dass die Infusionspumpe über die Datenschnittstellen selbst erfassen kann, mit welchen externen Komponenten sie gekoppelt ist, kann zum einen den Aufwand bei der Vorbereitung der Infusionsbehandlung gesenkt werden und weiterhin die Gefahr einer fehlerhaften Behandlung reduziert werden. Die Einleitung der Infusionsbehandlung kann vom Steuergerät eigenständig unterbrochen werden, wenn die erfassten Daten einen Fehler in der Vorbereitung der Behandlung zeigen, beispielsweise einen falsch angeschlossenen Medikamentenspeicher oder die Verwendung des falschen Therapieplans für einen Patienten.

Die Datenschnittstellen gestatten es darüber hinaus auch, während der laufenden Infusionsbehandlung deren Verlauf zu überwachen und ggf. die Infusionsbehandlung zu unterbrechen oder anzupassen, wenn dies aufgrund der erfassten Daten geboten erscheint.

Der Datenaustausch mit dem Patienten und mit dem Medikamentenspeicher erfolgt vorzugsweise zumindest abschnittsweise entlang der Fluidpfade, die durch die Fluideingänge und den Fluidausgang gebildet werden. Es wird daher als vorteilhaft angesehen, dass das mindestens eine Anschlussstück eine Verbindungseinrichtung zur Herstellung einer Datenverbindung mit einem gegensätzlichen Gegenanschlussstück an der Fluidleitung des Medikamentenspeichers oder des Patienten aufweist.

Eine solche Verbindungseinrichtung kann durch galvanisch koppelnde Kontaktflächen an den Anschlussstücken gebildet sein. Von besonderem Wert ist es jedoch, wenn die Anschlussstücke zu diesem Zweck mit einer Spule zur induktiven Datenübertragung versehen sind, insbesondere einer Spule mit zylindrischer oder konischer Grundform.

Durch das Koppeln der entsprechenden Anschlussstücke wird nicht nur eine Fluidverbindung geschaffen, sondern gleichzeitig auch eine Verbindung, über die Daten und/oder elektrische Energie übertragen werden kann.

Die Leitungseinheit kann im einfachsten Falle aus einer Schlauchstruktur mit einem Ausgang und zwei oder mehr Eingängen bestehen, an deren Schlauchenden die Anschlussstücke vorgesehen sind. Datenleitungen können entlang der Schlauchstruktur geführt sein.

Als besonders vorteilhaft angesehen wird jedoch eine Leitungseinheit, die über ein starres Tragteil verfügt, an dem die Fluidleitung vorgesehen oder befestigt ist. Dieses starre Tragteil wird bestimmungsgemäß als Ganzes mit der Basiseinheit gekoppelt, insbesondere werkzeuglos mittels einer Kopplungseinrichtung in Art eines Schnappverbinders. Durch die Anbringung des Tragteils an der Basiseinheit werden die Fluidleitungen in den Bereich des Pumpaktors und ggf. der Ventilaktoren gebracht, so dass nach der Ankoppelung der Fluidfluss durch die Leitungseinheit mittels der basiseinheitsseitigen Aktoren gesteuert werden kann.

Die Fluidleitungen der Leitungseinheit können auch bei Verwendung eines Tragteils in der schon beschriebenen Art durch eine Schlauchstruktur gebildet sein, insbesondere durch einheitliche Schlauchkörper mit mindestens zwei Enden, wobei an den Enden die Anschlussstücke vorgesehen sind. Die Schlauchstruktur kann jedoch durch Schnappverbinder an dem Tragteil festgeklemmt sein. Alternativ ist jedoch auch eine Gestaltung möglich, bei der die Fluidleitungen zumindest abschnittsweise unmittelbar durch Oberflächen des Tragteils gebildet werden. Diese Oberflächen sind somit in Flüssigkeitskontakt.

Das Tragteil ist vorzugsweise aus einem starren Kunststoff hergestellt. Insbesondere kommen hier Polycarbonat (PC), Polyvinylchlorid (PVC), Polyethylen (PE) und Polypropylen (PP) in Frage, aber auch andere Kunststoffe können Verwendung finden.

Die Fluidleitungen des Leitungsteils sind zu mindest abschnittsweise aus einem verformbaren Kunststoff gebildet, damit der Pumpaktor und ggf. die Ventilaktoren über Ventilblenden oder dergleichen in der Lage sind, den Leitungsquerschnitt zu reduzieren. Vorzugsweise kommen hier als Materialien für die verformbaren Teile der Fluidleitungen thermoplastische Polyurethane (TPU) und Thermoplastische Elastomere (TPE) in Frage. Weiterhin kann auch hierfür Polyvinylchlorid (PVC) verwendet werden. Auch Silikon ist als Werkstoff für die verformbaren Teile der Fluidleitungen gut geeignet.

Die genannten Anschlussstücke zum Anschließen eines patientenseitigen Schlauchs sowie der medikamentenspeicherseitigen Anschlussstücke sind im Falle einer Gestaltung mit Schlauchkörper vorzugsweise an dessen freien Enden vorgesehen. Insbesondere vorzugsweise sind die Anschlussstücke im Falle einer solchen Gestaltung zueinander und ggf. zum Tragteil nicht ortsfest, sondern über Eingangsschlauchabschnitte oder einen Ausgangsschlauchabschnitt beweglich. Vorzugsweise ist mindestens ein einen Fluideingang bildendes Anschlussstück an einem Eingangsschlauchabschnitt vorgesehen, wobei vorzugsweise eine mit der ersten Datenschnittstelle verbundene Datenleitung zumindest abschnittsweise entlang des Eingangsschlauchabschnitts geführt ist. Ebenso ist vorzugsweise an der Ausgangsseite das den Fluidausgang bildende Anschlussstück an einem Ausgangsschlauchabschnitt vorgesehen, wobei vorzugsweise eine mit der zweiten Datenschnittstelle verbundene Datenleitung zumindest abschnittsweise entlang des Ausgangsschlauchabschnitts geführt ist. Wenn eine solche Gestaltung vorgesehen ist, bei der Datenleitungen entlang einem Schlauchabschnitt geführt sind, so kann hierzu die Datenleitung außenseitig am Schlauchabschnitt befestigt sein oder auch als integraler Bestandteil in die Wandung des Schlauchabschnitts integriert sein.

Vorzugweise ist vorgesehen, dass Schlauchabschnitte, die im angekoppelten Zustand der Leitungseinheit an der Basiseinheit im Bereich des Pumpaktors oder des Ventilaktors angeordnet sind, frei von Datenleitungen sind, da die Verformung der Leitungswandung hier die Datenleitungen auf die Dauer verletzen könnten.

Bei einer zu den beweglichen Anschlussstücken alternativen Gestaltung ist vorgesehen, dass mindestens ein einen Fluideingang oder den Fluidausgang bildendes Anschlussstück ortsfest am Tragteil angebracht ist und somit nach Anbringung des Tragteils an der Basiseinheit ortsfest zu dieser angeordnet ist. Insbesondere vorzugsweise sind alle Fluideingänge und der Fluidausgang durch zueinander ortsfeste Anschlussstücke am Trageteil gebildet.

Die an der Leitungseinheit vorgesehenen Datenleitungen, die insbesondere vorzugsweise zu den jeweiligen Anschlussstücken geführt sind, sind mit ihrem anderen Ende mit der Basiseinheit und der dort vorgesehenen Steuereinrichtung verbunden. Vorzugsweise ist vorgesehen, dass zum Zwecke der Datenübertragung mindestens eine Anschlusseinrichtungzum lösbaren Anschließen an eine Gegen-Anschlusseinrichtung der Basiseinheit vorgesehen ist. Wird die Leitungseinheit an der Basiseinheit mechanisch angekoppelt, so wird hierbei vorzugsweise auch die Datenverbindung der Anschlusseinrichtung mit der Gegen-Anschlusseinrichtung hergestellt.

Insbesondere vorzugsweise verfügen die Anschlusseinrichtung und die Gegen-Anschlusseinrichtung über korrespondierende Kontaktflächen zur galvanischen Kopplung, wobei vorzugweise mindestens auf einer Seite Federkontakte vorgesehen sind. Alternativ sind auch nicht-galvanische Verbindungsarten zwischen der Basiseinheit und der Leitungseinheit möglich wie insbesondere eine induktive Verbindung.

Es kann eine einzige Anschlusseinrichtung an der Leitungseinheit und eine einzige Gegen-Anschlusseinrichtung an der Basiseinheit vorgesehen sein, wobei hierüber sowohl die Datenverbindungen zum Patienten als auch zu den Medikamentenspeichern geführt sein können. Alternativ dazu kann auch eine Mehrzahl von Anschlusseinrichtungen und Gegen-Anschlusseinrichtungen vorgesehen sein, insbesondere je eine Anschlusseinrichtung und Gegen-Anschlusseinrichtung für die Datenverbindungzum Patienten und fürdie Datenverbindungen zu den Medikamentenspeichern.

Wie eingangs bereits erwähnt, weist die Basiseinheit vorzugsweise nicht nur einen Pumpaktor auf, sondern auch mindestens einen elektrisch steuerbaren Ventilaktor, mittels dessen eine Ventileinrichtung geöffnet und geschlossen werden kann. Durch solche Ventileinrichtungen sind vorzugsweise alle Fluideingänge getrennt voneinander vom Fluidausgang trennbar. Vorzugsweise sind mindestens drei Fluideingänge und entsprechend mindestens drei Ventileinrichtungen vorgesehen. Auch dem Fluidausgang kann eine Ventileinrichtung zugeordnet sein, so dass insbesondere vor Trennung der Leitungseinheit von der Basiseinheit der Fluidausgang verschlossen wird.

Die Infusionspumpe weist vorzugsweise ein gemeinsames Pumpengehäuse auf, innerhalb dessen und/oder an dessen Vorderseite mindestens der mindestens eine Pumpaktor sowie mindestens eine Ventileinrichtung und insbesondere die Ventilaktoren angeordnet ist. Die Leitungseinheit kann an der Vorderseite des Gehäuses befestigt werden, um das Fördern der Flüssigkeit mittels der Pumpeinrichtung und das Öffnen und Schließen der Ventileinrichtungen zu ermöglichen.

Die Ventileinrichtungen können als bistabile Ventileinrichtungen ausgebildet sein, so dass die jeweilige Ventileinrichtung ohne Stromzufuhr in einem Schließzustand und einem Öffnungszustand verbleiben kann.

Demgegenüber bevorzugt wird allerdings üblicherweise eine Ausgestaltung der mindestens einen Ventileinrichtung als monostabile Ventileinrichtung. Eine solche monostabile Ventileinrichtung weist eine dem Ventilaktor entgegenwirkende Rückstellfeder auf, so dass die Ventileinrichtung ohne Stromzufuhr einen Schließzustand einnimmt. Sofern die Infusionspumpe aufgrund eines Defekts die Ventilaktoren nicht mehr bestromt, wird die entsprechende Ventileinrichtung entsprechend geschlossen.

Es ist vorgesehen, dass eine erfindungsgemäße Infusionspumpe über die erste und die zweite Datenschnittstelle mit mindestens einer medikamentenbezogenen Gegenstelle und einer patientenbezogenen Gegenstelle kommuniziert. Im Falle der patientenbezogenen Gegenstelle kann es sich insbesondere um einen integrierten Schaltkreis und/oder einen Speicher an einem Katheterdes Patienten handeln, mit dem das Steuergerät über diezweite Datenschnittstelle kommuniziert. Im Falle der medikamentenbezogenen Gegenstelle kann es sich insbesondere um einen integrierten Schaltkreis und/oder einen Speicher am Medikamentenspeicher handeln, mit dem das Steuergerät über die erste Datenschnittstelle kommuniziert.

Die Kommunikation kann insbesondere dem Zweck dienen, Steuerdaten zum Betrieb des Pumpaktors und/oder der Ventilaktoren hierüber in das Steuergerät einzulesen. Insbesondere kann vorgesehen sein, dass ein Therapieplan über eine der Datenschnittstellen eingelesen wird, der Informationen dazu enthält, welcher Patient welche Medikamente in welcher Reihenfolge zu erhalten hat. Eine Aktivierung des Pumpaktors und ein Schalten der Ventilaktoren findet dann gemäß diesem Therapieplan statt. Das Steuergerät ist vorzugsweise insbesondere dafür ausgebildet, den Therapieplan von einem angeschlossenen Medikamentenspeicher einzulesen.

Weiterhin kann die Datenkommunikation dem Zweck dienen, dass eindeutige Kennungen über die Datenschnittstellen eingelesen werden. Eine solche eindeutige Kennung kann dann anschließend genutzt werden, um den passenden Therapieplan auszuwählen. Dieser kann beispielsweise schon vorher in der Infusionspumpe hinterlegt sein oder auf Basis der eindeutigen Kennung, insbesondere einer Patientenkennung oder einer von der Patientenseite eingelesenen Therapiekennung, von einem externen Server eingelesen werden. Darüber hinaus dient das Einlesen von eindeutigen Kennungen, insbesondere von medikamentenbezogenen Kennungen, der Prüfung der Validität der jeweiligen Kennungen im Rahmen eines zuvor bereitgestellten Therapieplans. Zusätzlich kann das Steuergerät dafür ausgebildetsein, beim Anschluss eines Medikamentenspeichers über die erste Datenschnittstelle zu prüfen, ob der jeweilige Medikamentenspeicher, dessen Kennung zuvor eingelesen wurde, am hierfür vorgesehenen Anschlussstück angeschlossen worden ist.

Vorzugsweise gehtdie Überprüfung der Validität, insbesondere der Validität von über diezweite Datenschnittstelle eingelesenen Kennungen von Medikamentenspeichern, damit einher, die Echtheit des Medikamentenspeichers zu überprüfen. Dies kann durch Überprüfen einer auf diese Weise eingelesenen digitalen und vorzugsweise kryptographischen Authentifizierung erfolgen.

Die Datenschnittstellen können neben der Übertragung von Kennungen bzw. einem Therapieplan auch der Übertragung von Sensordaten dienen. Vorzugsweise ist ein Medikamentenspeicher mit entsprechender Sensorik ausgerüstet und das Steuergerät dafür ausgebildet, über die erste Datenschnittstelle Daten dieser Sensorik auszulesen, insbesondere die Temperatur oder optische Eigenschaften oder die Konzentration der Flüssigkeit.

Auch die Patientenseite, insbesondere ein patientenseitig vorgesehener Katheter, der über die zweite Datenschnittstelle an das Steuergerät angeschlossen ist, kann mit einer Sensorik versehen sein, deren Daten über die zweite Datenschnittstelle übertragen werden. Insbesondere kann die Sensorik einen Temperatursensor, einen Blutdrucksensor, einen Blutsauerstoffsensor und/oder einen Sensorzur Messung von Herzströmen oder zur Messung von Gewebeimpedanz umfassen, deren Daten vom Steuergerät ausgelesen werden. Die Sensorik dient dem Zweck, die Vitaldaten des Patienten zu überwachen. Insbesondere kann dadurch auch eine mögliche Änderung an den Vitaldaten in Reaktion auf die Infusionstherapie und ihre Teilphasen erkannt und ggf. dokumentiert werden.

Das Steuergerät kann dafür ausgebildet sein, den Pumpaktor und/oder einen Ventilaktor in Abhängigkeit der Sensordaten zu steuern. Insbesondere kann vorgesehen sein, dass das Steuergerät durch Stoppen des Pumpaktors oder durch Schließen von Ventileinrichtungen die Infusionsbehandlung unterbricht, wenn patientenseitige oder medikamentenspeicherseitige Sensordaten außerhalb eines definierten Wertebereichs liegen.

Darüber hinaus ist das Steuergerät vorzugsweise dafür ausgebildet, ein Warnsignal zu generieren und insbesondere in visueller oder akustischer Form abzugeben, wenn über die Datenschnittstellen eingelesene Daten eine von Sollparametern abweichende Situation repräsentieren, also beispielsweise ein falscher Medikamentenspeicher angeschlossen wird oder Sensoren kritische Daten liefern. Das Warnsignal kann als visuelles oder akustisches Warnsignal unmittelbar an der Infusionspumpe wahrnehmbar sein. Es kann sich jedoch auch oder zusätzlich um eine über ein Netzwerk versendete Wahrmitteilung handeln.

Das Steuergerät ist vorzugsweise dafür ausgebildet, über die Tätigkeit der Infusionspumpe ein Protokoll zu führen, also entsprechende Daten in einem eigenen Speicher abzulegen oder über eine Netzwerkschnittstelle oder eine der Datenschnittstellen zur Speicherung zu übertragen. Insbesondere wird es als vorteilhaft angesehen, wenn einige oder alle der folgenden Daten protokolliert werden: Anschließen eines Patienten an der Infusionspumpe, Anschließen eines Medikamentenspeichers an der Infusionspumpe, vorzugsweise einschließlich Speicherung von Daten über die Art des Medikaments und/oder umfassend eine eindeutige Kennzeichnung der Charge, Eingangswerte von mindestens einem Sensor, der einem Medikamentenspeicher oder dem Patienten zugeordnet ist.

Das Steuergerät der Infusionspumpe weist vorzugsweise zusätzlich zu den Datenschnittstellen eine Netzwerkschnittstelle auf, über die es mit einem externen Netzwerk verbunden ist. Diese Netzwerkschnittstelle kann insbesondere genutzt werden, um hierüber den patientenspezifischen Therapieplan und/oder Daten einer Patientenakte einzulesen, auf Basis derer dann die Validitätsprüfung in Hinblick auf angeschlossene Medikamentenspeicher erfolgen kann. Das Steuergerät kann insbesondere auch dafür ausgebildet sein, über die Netzwerkschnittstelle ein Protokoll über die Tätigkeit der Infusionspumpe zu versenden.

Vorzugsweise weist die Infusionspumpe eine Anzeigeeinrichtung, insbesondere in Art eines fest in die Infusionspumpe integrierten Displays, vorzugsweise eines Touch-Displays auf. Diese Anzeigeeinrichtung kann primär dem Zweck dienen, während der Vorbereitung der Infusionspumpe wesentliche Informationen anzuzeigen, so beispielsweise Daten zu den über die erste und die zweite Datenschnittstelle angeschlossenen Gegenstellen auf Patientenseite und auf Seite der Medikamentenspeicher. Die Anzeigeeinrichtung kann im Falle einer Gestaltung, bei der das Steuergerät zum Auslesen von Sensordaten ausgebildet ist, auch dem Zweck dienen, diese Sensordaten anzuzeigen. Weiterhin kann die Anzeigeeinrichtung dem Zweck dienen, Informationen zu einer derzeit ablaufenden Infusionsbehandlung in für medizinisches Fachpersonal oder den Patienten geeigneter Form darzustellen. Insbesondere kann angezeigt werden, wie weit die Infusionsbehandlung fortgeschritten ist und welches Medikament zum gegenwärtigen Zeitpunkt gefördert wird.

Von besonderem Vorteil ist es, wenn die Infusionspumpe mit ihrer Anzeigeeinrichtung für ergänzende Funktionen genutzt wird, die nicht unmittelbar die Durchführung der Therapie mittels der Infusionspumpe dienen. Zu solchen Funktionen gehört die Nutzung der Infusionspumpe als Feedback-System. Die Infusionspumpe ist demnach vorzugsweise dafür ausgebildet, ein Feedback des Patienten entgegenzunehmen, beispielsweise in Form einer Bedienung von Bedienelementen und/oder von einem Touchscreen der Infusionspumpe. Der Patient kann hierüber beispielsweise sein subjektiv empfundenes aktuelles Befinden eingeben. Gegebenenfalls kann dies auch genutzt werden, um in Reaktion auf ein solches Feedback die Infusionsbehandlung zu unterbrechen oder anzupassen. Das eingegebene Feedback kann, gegebenenfalls ergänzt um Daten zur aktuell laufenden Infusionsbehandlung und/oder von der Infusionspumpe eingelesenen Sensordaten über die beschriebenen Netzwerkschnittstelle versendet werden.

Das Vorhandensein einer Anzeigeeinrichtung kann zusammen mit einer in die Infusionspumpe integrierten Kamera auch genutzt werden, um Videotelefonate durchzuführen. Insbesondere kann der Patient hierüber Kontakt zu medizinischem Personal aufnehmen.

Die Erfindung betrifft nicht nur die Infusionspumpe selbst, sondern darüber hinaus auch eine Infusionsanordnung, die neben der Infusionspumpe mindestens einen mit einem flüssigen Medikament befüllten Medikamentenspeicher aufweist. Dieser ist über die erste Datenschnittstelle mit dem Steuergerät der Infusionspumpe verbunden. Der Medikamentenspeicher ist mit einer Gegenstelle in Form mindestens eines integrierten Schaltkreises oder eines elektronischen Speichers versehen, dessen Daten vom Steuergerät ausgelesen werden können. Vorzugsweise weist der Medikamentenspeicher einen Verbindungsschlauch zum Anschluss an der Infusionspumpe auf, an dessen Ende ein Gegen-Anschlussstück zum Fluideingang-Anschlussstück der Infusionspumpe vorgesehen ist. Ebenso wie das Anschlussstück an der Infusionspumpe weist auch das Gegen-Anschlussstück vorzugsweise eine Verbindungseinrichtung zur Herstellung der Datenverbindung auf, insbesondere in Form einer Spule. Zusätzlich zum integrierten Schaltkreis und/oder dem Speicher oder alternativ hierzu kann am Medikamentenspeicher auch mindestens ein Sensor vorgesehen sein, insbesondere ein Temperatursensor, ein Sensor zur Erfassung von optischen Eigenschaften der Flüssigkeit oder ein Sensor zur Erfassung der Konzentration der Flüssigkeit. Die Sensordaten dieses Sensors können von dem Steuergerät über die erste Datenschnittstelle ausgelesen werden.

Weiterhin weist die Infusionsanordnung vorzugsweise eine patientenseitige Zugangseinrichtung auf, die über die zweite Datenschnittstelle mit dem Steuergerät der Infusionspumpe verbunden ist. Ebenso wie der Medikamentenspeicher kann die Zugangseinrichtung, die insbesondere einen Katheter umfasst, über einen integrierten Schaltkreis und/oder einen elektronischen Speicher verfügen, auf die das Steuergerät der Infusionspumpe über die zweiten Datenschnittstelle zugreifen kann. Vorzugsweise ist der Zugangseinrichtung weiterhin mindestens ein Sensor zugeordnet, der über die zweite Datenschnittstelle durch das Steuergerät auslesbar ist. Es handelt sich insbesondere vorzugsweise im einen Temperatursensor, einen Blutdrucksensor, einen Sauerstoffsensor zur Messung des Sauerstoffgehalts im Blut des Patienten oder um einen Sensor zur Messung von Herzströmen oder zur Messung von Gewebeimpedanz.

Die beschriebene Infusionsanordnung ist vorzugsweise Teil eines erfindungsgemäßen Infusionssystems. Ein solches Infusionssystem umfasst neben der Infusionsanordnung einen Server, der über ein Netzwerk mit dem Steuergerät der Infusionspumpe verbunden ist. Vorzugsweise umfasst das Infusionssystem eine Mehrzahl von Infusionspumpen, die jeweils mit dem gleichen Server verbunden sind. Der Server hat vorzugsweise über das Netzwerk Zugriff auf Patientenakten und/oder Therapiepläne.

Das Steuergerät der Infusionspumpe ist dafür ausgebildet, über das Netzwerk Daten an den Server zu übermitteln oder von dort abzurufen. Insbesondere kann das Steuergerät dafür ausgebildet sein, Daten zu einer Therapie vom Server abzurufen, wobei die hierauf gerichtete Anfrage vorzugsweise unter Nutzung von Daten erstellt wird, die vom Steuergerät über die erste oder zweite Datenschnittstelle eingelesen wurden, also insbesondere einer Kennung, die dem Patienten eindeutig zugeordnet ist. Weiterhin kann das Steuergerät dafür ausgebildet sein, Statusmeldungen oder das oben beschriebenen Patienten-Feedback an den Server zu übertragen, vorzugsweise in Echtzeit. Die oben erwähnte Protokollierung der Infusionsbehandlung kann dann statt durch die Infusionspumpe selbst durch den Server erfolgen.

Neben der Infusionspumpe, der Infusionsanordnung und dem Infusionssystem betrifft die Erfindung auch ein korrespondierendes Verfahren zum Betrieb einer Infusionspumpe, die vorzugsweise gemäß obiger Beschreibung ausgebildet ist.

Das Verfahren sieht vor, dass die Infusionspumpe Daten von einem Medikamentenspeicher über die erste Datenschnittstelle einliest und/oder Daten über den Patienten über die zweite Datenschnittstelle einliest. Es erfolgt dann eine Verarbeitung der Daten, insbesondere zur Prüfung der Validität der Daten des Patienten und des Medikamentenspeichers, wobei im Zuge der Prüfung insbesondere die Echtheit eines angeschlossenen Medikamentenspeichers oder dessen Kompatibilität mit dem Therapieplan geprüft wird und/oder wobei geprüft wird, ob der Medikamentenspeicher am dafür vorgesehenen Fluideingang angeschlossen ist. Die Verarbeitung der Daten kann weiterhin das Einlesen von Therapiedaten über eine Netzwerkschnittstelle umfassen.

Als Reaktion dieser Datenverarbeitung wird, üblicherweise nach manueller Freigabe durch medizinisches Personal, die Flüssigkeitsförderung mittels der Infusionspumpe gestartet und/oder es wird mindestens eine Ventileinrichtung geöffnet, wenn die eingelesen Daten der Infusionsbehandlung nicht entgegenstehen. Anderenfalls wird die Infusionsbehandlung nicht gestartet, insbesondere indem der Pumpaktor deaktiviert oder nicht aktiviert wird oder indem ein Ventilaktor eine Ventileinrichtung der Infusionspumpe schließt.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt eine Infusionspumpe als Teil eines Infusionssystems.
Fig. 2 und 3 zeigen eine Basiseinheit und eine Leitungseinheit der Infusionspumpe.
Fig. 4 und 5 zeigen alternative Gestaltungen der Leitungseinheit der Infusionspumpe.
Fig. 6 zeigt eine Anschlusseinrichtung der Infusionspumpe.
Fig. 7 und 8 zeigen den Aufbau von Ventileinrichtungen der Infusionspumpe.

### DETAILLIERTE BESCHREIBUNG DER AUSFÜHRUNGSBEISPIELE

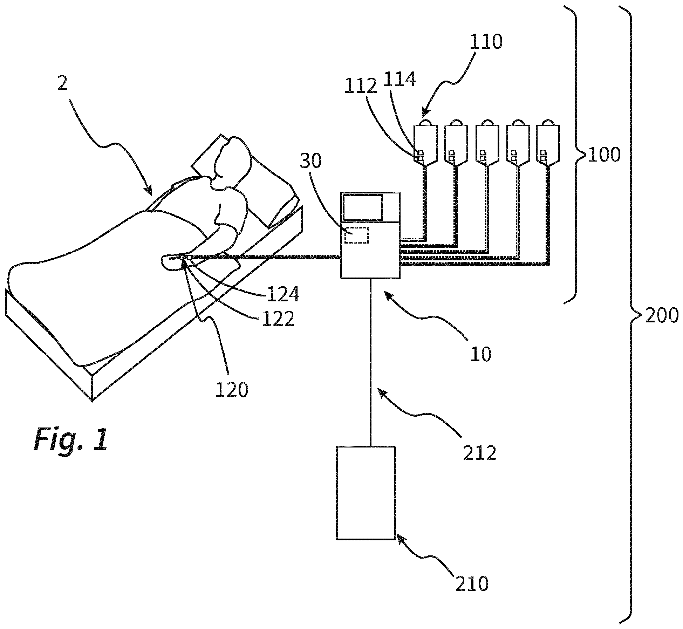

Fig. 1 zeigt eine Infusionspumpe 10, die Teil einer Infusionsanordnung 100 ist.

Die Infusionspumpe 10 dient dem primären Zweck, Flüssigkeitaus den Medikamentenspeichern 110 einem Patienten 2 zuzuführen. Der Patient 2 ist über eine Zugangseinrichtung 120 an einen Fluidausgang der Infusionspumpe 10 angeschlossen. Die im Beispiel insgesamt fünf Medikamentenspeicher 110 enthalten unterschiedliche Medikamente, die üblicherweise spezifisch für den Patienten 2 erstellt wurden. Einer der Medikamentenspeicher 110 kann eine Spülflüssigkeit enthalten, um die Fluidleitungen und die Infusionspumpe 10 zwischen unterschiedlichen Phasen der Medikamentenabgabe zu spülen.

Die Zugangseinrichtung 120 sowie die Medikamentenspeicher 110 sind nicht ausschließlich fluidisch mit der Infusionspumpe 10 verbunden, sondern darüber hinaus über Datenleitungen 65A-65F. Über diese Datenleitungen ist ein Steuergerät 30 der Infusionspumpe 10 mit Gegenstellen auf Patientenseite bzw. Medikamentenspeicherseite verbunden.

Im Falle des Patienten weist die Zugangseinrichtung 120 einen Speicher 122 auf. Zusätzlich kann hier ein Prozessor zur Verarbeitung der Daten vorgesehen sein. Weiterhin ist auf der Seite der Zugangseinrichtung 120 vorzugsweise ein Sensor 124 oder eine Mehrzahl von Sensoren 124 vorgesehen. Diese können unmittelbar integraler Teil der Zugangseinrichtung 120 sein oder mit der Zugangseinrichtung über weitere Leitungen verbunden sein.

Der Speicher 122 enthält vorzugsweise zumindest ein für den Patienten oder die geplante Infusionstherapie eindeutiges Kennzeichen, beispielsweise eine ID-Nummer. Es können jedoch auch darüber hinausgehende Nutzdaten hier abgelegt sein, beispielsweise der Therapieplan selbst.

Die Sensoren dienen insbesondere der Überprüfung von Vitalfunktionen des Patienten 2. Es kann sich beispielweise um einen Temperatursensor, einen Blutdrucksensor, oder einen Blutsauerstoffsensor handeln. Auch Sensoren zur Messung von Herzströmen und zur Messung von Gewebeimpedanz können vorgesehen sein. Weiterhin kann es sich auch um Sensoren handeln, die der Überprüfung der Funktionsweise der Infusionsanordnung dienen. Beispielsweise kann Sensorik zur Prüfung des Fluidstroms hier vorgesehen sein, beispielsweise zur Prüfung, ob es eine fluidische Unterbrechung zwischen der Infusionspumpe und der Zugangseinrichtung gibt.

Auf der Seite der Medikamentenspeicher 110 ist im Falle der Gestaltung der Fig. 1 auch jeweils ein Speicher 112 sowie mindestens ein Sensor 124 vorgesehen.

Der Speicher 112 enthält vorzugsweise ein eindeutiges Kennzeichen, welches für den konkreten Medikamentenspeicher eindeutig ist. Zusätzlich oder stattdessen können auch Informationen zu der enthaltenen Flüssigkeit hier abgelegt sein, so insbesondere deren Zusammensetzung, ein Kennzeichen des Patienten, für den die Flüssigkeit erstellt wurde, oder auch das Datum der Erstellung und/oder ein Ablaufdatum.

Des Weiteren kann der Speicher 112 auch einen Therapieplan enthalten. Dieser Therapieplan kann neben Informationen dazu, wie die im betreffenden Medikamentenspeicher selbst enthaltene Flüssigkeit abzugeben ist, auch Informationen über weitere Medikamente enthalten, mit denen gemeinsam die Flüssigkeit abzugeben ist.

Der Sensor 114 dient der Überwachung des Medikamentenspeichers 110 und insbesondere der darin enthaltenen Flüssigkeit. Der Sensor 114 kann ein Temperatursensor, ein Sensor zur Erfassung von optischen Eigenschaften der Flüssigkeit oder ein Sensor zur Erfassung der Konzentration der Flüssigkeit sein. Auch ein Sensor 114 zur Erfassung des Füllstandes des Medikamentenspeicher kann zweckmäßig sein.

Das Steuergerät 30 ist in der Lage, über die Datenleitungen 65A-65F auf die Speicher 112, 122 lesend und ggf. auch schreibend zuzugreifen. Das Steuergerät 30 kann stattdessen auch mit Gegenstellen an den Medikamentenspeichern 110 und der Zugangseinrichtung 120 Daten austauschen, wobei diese Gegenstellen den Zugriff auf die jeweiligen Speicher 112,122 und Sensoren 114, 124 gewähren.

Darüber hinaus kann vorgesehen sein, dass das Steuergerät 30 über ein Netzwerk 212 auf einen zugeordneten Server 210 zugreift, um von hier Daten zu erhalten, beispielsweise einen Therapieplan, oder um Daten zum Server zu schicken, beispielsweise Statusdaten, die eine serverseitige Erstellung eines Therapieprotokolls ermöglichen. Stattdessen kann das Therapieprotokoll auch lokal durch das Steuergerät 30 erzeugt werden und zumindest zunächst in dessen Speicher abgelegt werden.

Das Steuergerät 30 steuert die Flüssigkeitszufuhr aus den Medikamentenspeichern 110 zum Patienten 2. In nachfolgend noch näher erläuterter Weise ist das Steuergerät 30 dafür ausgebildet, über Aktoren die Flüssigkeitsförderung zu steuern, insbesondere die Medikamentenspeicher 110 selektiv freigeben und trennen zu können sowie Flüssigkeit aus dem freigegebenen Medikamentenspeicher 110 zur Zugangseinrichtung 120 zu fördern.

Das Steuergerät 30 ist dafür ausgebildet, die Steuerung der Flüssigkeitszufuhr gemäß einem für den Patienten spezifisch zusammengestellten Therapieplan durchzuführen. DieserTherapieplan enthält die erforderlichen Informationen dazu, in welcher Reihenfolge und welcher Menge welche Medikamente abzugeben sind.

Der Therapieplan kann auf verschiedene Arten und Weisen in das Steuergerät gelangen. Er kann unmittelbar an der Infusionspumpe über dortige Eingabemittel eingegeben werden oder von einem separaten Speicher eingelesen werden.

Im Rahmen der vorliegenden Erfindung wird der Therapieplan jedoch vorzugsweise unmittelbar aus dem Speicher 122 auf Patientenseite oder aus dem Speicher 112 eines der Medikamentenspeicher eingelesen. Alternativ kann der Therapieplan auf Basis einer Kennzeichnung, die aus einem der Speicher ausgelesen wurde oder auf Basis einer manuellen Auswahl an der Infusionspumpe 10 über das Netzwerk 212 vom Server 210 bezogen werden.

Der Therapieplan enthält Informationen darüber, für welchen Patienten er erstellt wurde und welche Medikamente im Rahmen der Therapie Verwendung finden. Das Steuergerät 30 ist in der Lage, diese Vorgaben mit Daten abzugleichen, die von den unterschiedlichen Medikamentenspeichern 110 sowie von der Zugangseinrichtung 120 eingelesen worden sind.

So kann insbesondere geprüft werden, ob die angeschlossenen Medikamentenspeicher 110 mit den im Therapieplan enthaltenen übereinstimmen und dass alle erforderlichen Medikamentenspeicher 110 angeschlossen worden sind. Es kann auch geprüft werden, ob die Medikamentenspeicher 110 an den richtigen Anschlussstücken der Infusionspumpe 10 angeschlossen sind.

Weiterhin kann geprüft werden, ob der richtige Patient 2 bzw. die Zugangseinrichtung 120 des richtigen Patienten 2 an die Infusionspumpe angeschlossen ist.

Sind alle Voraussetzungen gemäß Therapieplan erfüllt, so startet das Steuergerät 30 die Therapie, indem in Übereinstimmung mit dem Therapieplan ein erster Medikamentenspeicher 110 über eine Ventileinrichtung geöffnet wird, während die anderen geschlossen bleiben. Durch Aktivierung einer Pumpeinrichtung wird die Flüssigkeit aus dem geöffneten Medikamentenspeicher 110 zurZugangseinrichtung 120 gefördert und an den Patienten abgegeben.

Sobald die gemäß Therapieplan vorgesehene Flüssigkeitsmenge des ersten Medikaments erreicht wurde, wird durch das Steuergerät die entsprechende Ventileinrichtung geschlossen und eine Ventileinrichtung eines Medikamentenspeichers 110 geöffnet, der mit einer Spülflüssigkeit befüllt wird. Nachdem hiermit die Fluidleitungen gespült wurden, wird die Ventileinrichtung eines anderen Medikamentenspeichers 110 geöffnet und die Infusionstherapie mit der entsprechenden Flüssigkeit fortgesetzt.

Während des Ablaufs der Infusionsbehandlungüberwacht das Steuergerät 30die Sensoren 114,124. Auf Seiten der Medikamentenspeicher kann insbesondere überwacht werden, ob die jeweiligen Flüssigkeiten die gewünschte Temperatur aufweisen. Auf Seiten des Patienten werden vor allem dessen Vitaldaten überwacht.

Sofern die vom Steuergerät 30 erfassten Daten eine potentiell kritische Situation erkennen lassen, kann das Steuergerät verschiedene Maßnahmen treffen. Es kann die Therapie zunächst vollständig unterbrechen, insbesondere durch Deaktivierung der Pumpeinrichtung der Infusionspumpe 10. Es kann stattdessen auch die Therapie anpassen. Weiterhin kann vorgesehen sein, dass das Steuergerät 30 den Server 210 oder anderweitige externe Komponenten betreffend den erkannten Sachverhalt kontaktiert, so dass beispielsweise medizinisches Fachpersonal informiert werden kann.

Über eine Anzeigeeinrichtung 34 der Infusionspumpe 10 kann nicht nur das medizinische Fachpersonal die Infusionspumpe 10 bedienen. Es kann zusätzlich vorgesehen sein, dass der Patient 2 selbst die Anzeigeeinrichtung und zugehörige Bedienelemente nutzt, um ein Feedback zu geben. So kann der Patient unmittelbar medizinisches Personal heranrufen oder ein Videotelefonat mit einem Mediziner über die Anzeigeeinrichtung 34 initiieren, falls er dies für geboten hält.

Er kann stattdessen aber auch auf dem Bildschirm innerhalb eines hierfür vorgesehenen DialogsystemsAuskunft übersein Befinden geben. Das Steuergerät 30 kann dann ggf. unter Einbeziehung solcher Daten entscheiden, ob die Infusionsbehandlung unterbrochen werden sollte.

Vorzugsweise ist das Steuergerät 30 dafür ausgebildet, die Infusionsbehandlung zu protokollieren und entsprechende Daten abzulegen, wobei die Daten sowohl lokal in der Infusionspumpe 10 oder aber über das Netzwerk 212 auf einem Server 210 abgelegt werden können. Insbesondere legt das Steuergerät 30 Daten zu den jeweils erfolgen Therapieschritten und den jeweiligen Zeitpunkt der Schritte sowie Daten der Sensoren 114, 124 ab. Insbesondere kann dieses Therapieprotokoll unmittelbar in der Patientenakte des jeweiligen Patienten 2 abgelegt werden.

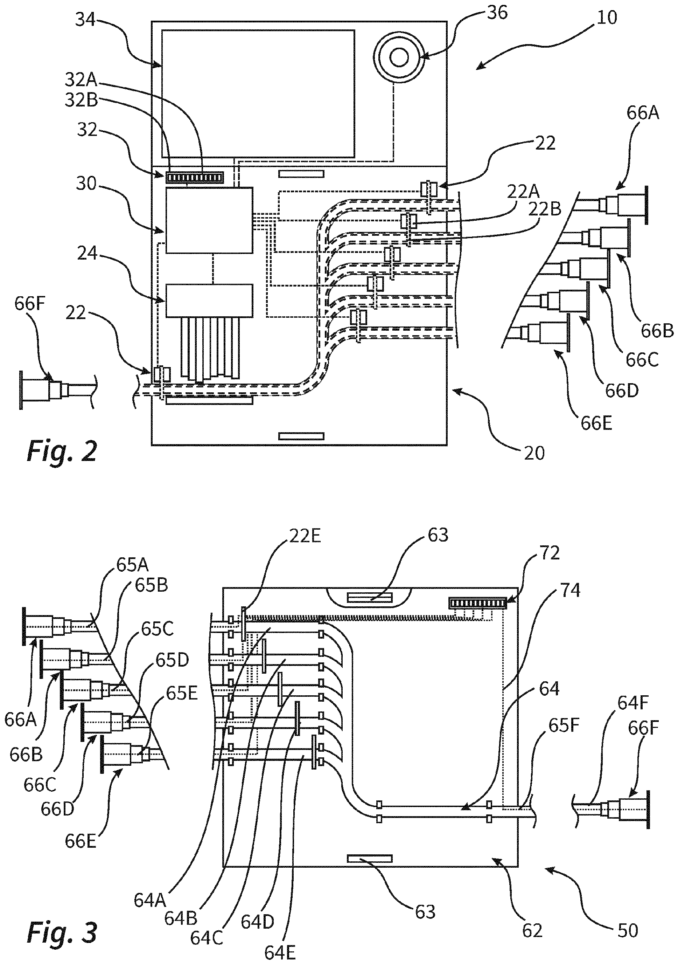

Fig. 2 und 3 zeigen ein erstes Ausführungsbeispiel der Infusionspumpe 10. Die Infusionspumpe 10 verfügt über zwei Hauptbaugruppen, nämlich eine Basiseinheit 20, die in Fig. 2 dargestellt ist, und eine Leitungseinheit 50, die in Fig. 3 dargestellt ist.

Die Basiseinheit 20 weist ausschließlich nicht unmittelbar flüssigkeitsführende Komponenten auf, insbesondere ein Gehäuse, innerhalb dessen bzw. an dessen Vorderseite verschiedene Aktoren vorgesehen sind, nämlich ein Pumpaktor 40 sowie fünf eingangsseitige und einen ausgangsseitigen Ventilaktor 22A als Teil von Ventileinrichtungen 22. Diese Aktoren sind am Steuergerät 30 angeschlossen, so dass das Steuergerät 30 hierüber die Flüssigkeitsförderung steuern kann.

Weiterhin weist die Basiseinheit die schon beschriebene Anzeigeeinrichtung 34 auf, insbesondere ausgestaltet als Touch-Display. Auch ein Lautsprecher 36 ist in die Basiseinheit integriert.

Die Fluidführung erfolgt vollständig in der Leitungseinheit 50. Die Leitungseinheit 50 verfügt über eine Tragteil 62 aus einem starren Kunststoff. An diesem Tragteil ist eine Schlauchstruktur 52 vorgesehen, die die Fluidleitung64 bildet. Diese Schlauchstruktur 52 ist mittels Schnappverbindern 54 am Tragteit 62 befestigt. Die Schlauchstruktur verfügt über fünf eingangsseitige Schlauchabschnitte, an denen jeweils ein Anschlussstück 66A-66E vorgesehen ist. Die Schlauchstruktur verfügt weiterhin über einen ausgangsseitigen Schlauchabschnitt, an dem ein weiteres Anschlussstück 66F angebracht ist. Die Anschlussstücke sind jeweils in Art eines Luer-Konnektors gemäß der Normenfamilie ISO 80369 ausgebildet.

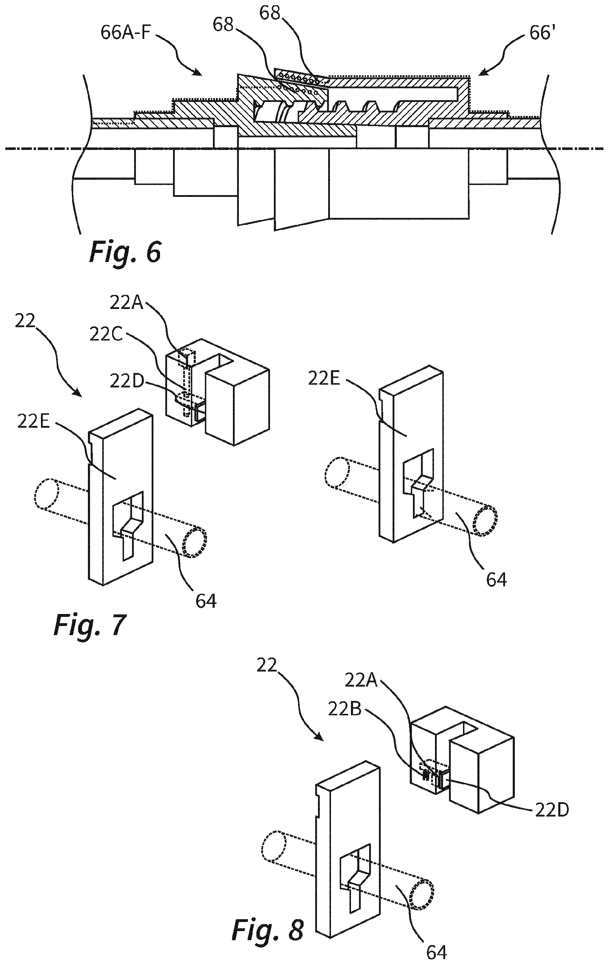

Ein Anschlussstück entsprechend der Anschlussstücke 66A-66F ist nochmals in Fig. 6 dargestellt, verbunden mit einem Gegen-Anschlussstück 66'. Die Zuordnung ist hier willkürlich, denn auch das rechtsseitige Anschlussstück gemäß Fig. 6 könnte einem oder mehreren der Anschlussstücke an der Leitungeinheit entsprechen.

Wie in Fig. 6 ersichtlich ist, weisen die Anschlussstücke 66A-66F Spulen 68 als Verbindungseinrichtung auf, um Daten und/oder elektrische Leistung über die verbundenen Anschlussstücke 66A-66F hinweg übertragen zu können. Die Spulen sind als zylindrische oder konische Spulen geformt, könnten aber auch in Art von planaren Spulen ausgebildet sein.

Die an den Anschlussstücken 66A-66F und deren Spulen 68 angeschlossenen Datenleitungen 65A-65F sind abschnittsweise entlang der Schlauchstruktur 52 geführt und dann im Bereich des Tragteils 62 zu einer gemeinsamen Anschlusseinrichtung 72 geführt.

Die Leitungseinheit 50 weist eine Kopplungseinrichtung 63 zur mechanischen Ankopplung an die Basiseinheit 20 auf. Bestimmungsgemäß wird die Leitungseinheit 50 der Fig. 3 von vorne gegen die Basiseinheit 20 gedrückt, so dass die Kopplungseinrichtungen mit einer korrespondierenden Geometrie auf Seiten der Basiseinheit lösbar verschnappt.

Neben dieser mechanischen Kopplung gelangt dabei die Fluidleitung 64 auch in den Bereich des Pumpaktors 24, so dass dieser die Flüssigkeit durch die flexible Wandung der Fluidleitung 64 in Richtung des Fluidausgangs fördern kann. An den insgesamt fünf Eingangszweigen der Fluidleitung 64 sowie auf der Ausgangsseite der Fluidleitung 64 sind jeweils Ventilblenden 22E vorgesehen. Diese Ventilblenden 22E gelangen beim Aufsetzen der Leitungseinheit 50 auf die Basiseinheit 20 in Eingriff mit den korrespondierenden Ventilaktoren 22A.

Die Ventileinrichtungen 22 mitsamt der Ventilblende 22E sind in den Fig. 7 und 8 dargestellt.

Bei der Gestaltung der Fig. 7 handelt es sich um eine bistabile Ventileinrichtung. Auf Seite der Basiseinheit 20 ist eine Aufnahme vorhanden, in die die Ventilblende 22E eingeschoben wird. Innerhalb dieser Aufnahme ist ein Kopplungsfortsatz 22D vorgesehen, der mit einer Nut in der Ventilblende 22E in Eingriff gelangt, so dass der als Stellmotor ausgebildete Ventilaktor 22A über eine Spindel 22C den Kopplungsfortsatz 22D und damit die Ventilblende 22E relativ zur Fluidleitung verschiebt. So kann der geschlossene Zustand hergestellt werden, der in Fig. 7 auf der rechten Seite dargestellt ist.

Bei der Gestaltung der Fig. 8 handelt es sich um eine monostabile Ventileinrichtung 22. Der Grundaufbau ist hier ähnlich der Fig. 7. Der Unterschied besteht darin, dass hier eine Rückstellfeder 22B vorgesehen ist, durch die der Kopplungsfortsatz 22D nach oben kraftbeauschlagtwird. Die Ventileinrichtung schließt daher für den Fall, dass kein Strom an der Ventileinrichtung 22 anliegt. Wird die Ventileinrichtung 22 bestromt, so wird hierdurch ein Elektromagnet aktiviert, der den Ventilaktor 22A darstellt. Dieser Elektromagnet zieht den Kopplungsfortsatz 22D und damit die Ventilblende 22E nach unten, so dass hierdurch die Ventileinrichtung 22 geöffnet wird.

Die Anbringung der Leitungseinheit 50 an der Basiseinheit 20 führt darüber hinaus auch zur Ankopplung der Anschlusseinrichtung 72 an der Leitungseinheit an der Gegenanschlusseinrichtung 32 der Basiseinheit 20. Somit werden die Anschlussstücke 66A-66F, die Teil der Leitungseinheit 50 sind, mit dem Steuergerät 30 verbunden, so dass die oben beschriebene Kommunikation mit an den Anschlussstücken 66A-66F angeschlossenen Gegenstellen erfolgen kann.

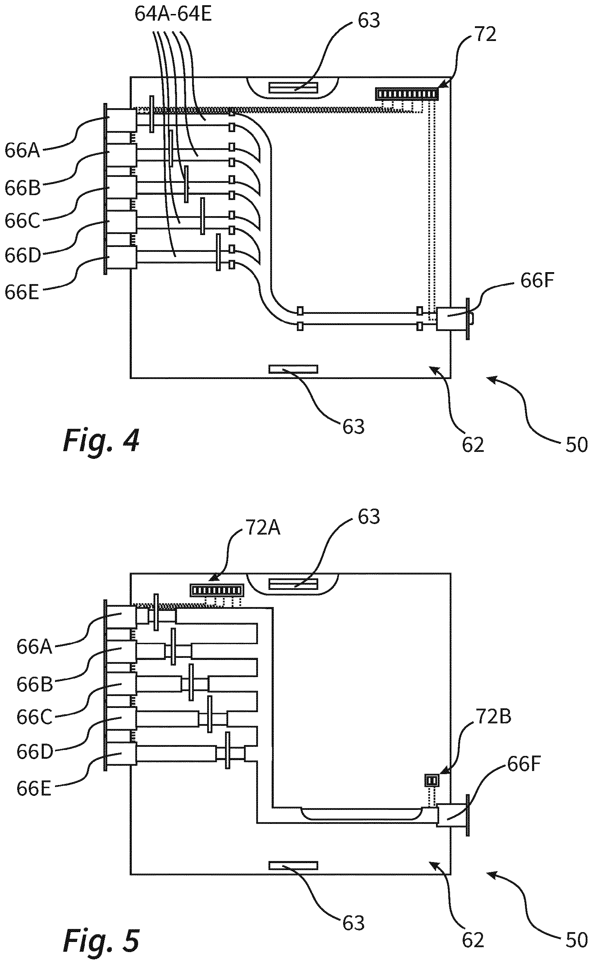

Die Fig. 4 und 5 zeigen alternative Gestaltungen der Leitungseinheit 50.

In beiden Fällen ist abweichend von der Gestaltung der Fig. 3 vorgesehen, dass die Anschlussstücke 66A bis 66F nicht an Schlauchabschnitten vorgesehen sind, die gegenüber dem Tragteil 62 beweglich sind. Stattdessen sind die Anschlussstücke 66A bis 66F jeweils ortsfest am Tragteil angebracht.

Bei der Gestaltung der Fig. 5 ist zusätzlich abweichend von den Gestaltungen der Fig. 3 und 4 vorgesehen, dass die Fluidleitungen 64 nicht durch eine Schlauchstruktur 52 gebildet ist, die am Tragteil 62 befestigt ist. Stattdessen sind die Fluidleitungen 64 hier unmittelbar durch das Tragteil 62 gebildet. Die Fluidleitungen 64 werden also zum überwiegenden Teil durch starre Wandungen des Tragteils 62 begrenzt. Nur in Teilabschnitten 64A, 64B ist die Fluidleitungen mit einer formflexiblen Wandung versehen, so dass hier der Pumpaktor 24 auf die Fluidleitung 64 fördernd wirken kann und die Ventilblende 22E die Fluidleitung selektiv verschließen kann.

## Patentansprüche

1. Infusionspumpe (10) zur Versorgung eines Patienten (2) mit mindestens einem Medikament in flüssiger Form mit den folgenden Merkmalen:
a. die Infusionspumpe (10) weist eine Basiseinheit (20) auf, und
b. die Infusionspumpe (10) weist eine an die Basiseinheit (20) zu koppelnde Leitungseinheit (50) auf, die eine zumindest abschnittsweise formflexible Fluidleitung (64) mit mindestens einem einen Fluideingang bildenden Anschlussstück (66A-66E) und mit einem einen Fluidausgang bildenden Anschlussstück (66F) umfasst, und
c. die Infusionspumpe (10) weist an der Basiseinheit (20) mindestens einen elektrisch betriebenen Pumpaktor (24) auf, der dafür ausgebildet ist, zwischen dem Fluideingang und dem Fluidausgang von außen auf die zumindest abschnittsweise formflexible Fluidleitung (64) zu wirken und hierdurch das Medikament aus mindestens einem am mindestens einen Fluideingang (66A-66E) angeschlossenen Medikamentenspeicher (110) zum Fluidausgang und damit zum Patienten (2) zu fördern, und
d. die Infusionspumpe (10) weist an der Basiseinheit (20) ein Steuergerät (30) zur Steuerung mindestens des Pumpaktors (24) auf, wobei das Steuergerät (30) mit mindestens einerersten Datenschnittstelle (32A) zur Verbindung mit dem mindestens einen Medikamentenspeicher (110) und mit einer zweiten Datenschnittstelle (32B) zur Verbindung mit dem Patienten verbunden ist, und
e. das Steuergerät (30) ist dafür ausgebildet, patientenbezogene und medikamentenbezogene Daten über die Datenschnittstellen (32A, 32B) zu erfassen und mindestens den Pumpaktor (24) in Abhängigkeit der über die Datenschnittstellen (32A, 32B) erfassten Daten zu steuern.

2. Infusionspumpe (10) nach Anspruch 1 mit dem folgenden weiteren Merkmal:
a. das mindestens eine Anschlussstück (66A-66F) weist eine Verbindungseinrichtung (68) zur Herstellung einer Datenverbindung mit einem gegensätzlichen Gegenanschlussstück (66') auf, insbesondere in Form einer Spule (68) für induktive Datenübertragung.

3. Infusionspumpe (10) nach Anspruch 1 oder 2 mit dem folgenden weiteren Merkmal:
a. Die Leitungseinheit (50) weist ein starres Tragteil (62) auf, an dem die Fluidleitung (64) vorgesehen oder befestigt ist,
vorzugsweise mit mindestens einem der folgenden zusätzlichen Merkmale:
b. die Fluidleitung (64) istzumindestabschnittsweise durch eine Oberfläche des Tragteils (62) gebildet, oder
c. die Fluidleitung (64) ist durch einen einheitlichen Schlauchkörper mit mindestens zwei Enden gebildet, wobei an den Enden die Anschlussstücke (66A-66F) vorgesehen sind, und/oder
d. das Tragteil (62) weist eine Kopplungseinrichtung (63) zur lösbaren Befestigung des Tragteils (62) an der Basiseinheit (20) auf, und/oder
e. das Tragteil (62) ist zumindest überwiegend aus einem starren Kunststoff hergestellt, insbesondere aus Polycarbonat (PC), Polyvinylchlorid (PVC), Polyethylen (PE) und/oder Polypropylen (PP), und/oder
f. die Fluidleitung ist zumindest abschnittsweise aus einem elastisch verformbaren Kunststoff, vorzugweise aus einem thermoplastischen Polyurethan (TPU), einem thermoplastische Elastomere (TPE), oder einem Gummi, vorzugsweise Silikon, hergestellt.

4. Infusionspumpe (10) nach Anspruch 3 mit mindestens einem derfolgenden weiteren Merkmale:
a. mindestens ein einen Fluideingang bildendes Anschlussstück (66A-66E) ist ortsfest am Tragteil (62) vorgesehen, und/oder
b. das den Fluidausgang bildenden Anschlussstück (66F) ist ortsfest am Tragteil vorgesehen, vorzugsweise mit dem folgenden zusätzlichen Merkmal:
c. alle Anschlussstücke (66A-66F) sind ortsfest am Tragteil (62) vorgesehen.

5. Infusionspumpe (10) nach einem der vorstehenden Ansprüche mit mindestens einem der folgenden weiteren Merkmale:
a. mindestens ein einen Fluideingang bildendes Anschlussstück (66A-66E) ist an einem Eingangsschlauchabschnitt (64A-64E) vorgesehen, wobei eine mit der ersten Datenschnittstelle (32A) verbundene Datenleitung (65A-E) zumindest abschnittsweise entlang des Eingangsschlauchabschnitts (64A-64E) geführt ist, und/oder
b. das den Fluidausgang bildende Anschlussstück (66F) ist an einem Ausgangsschlauchabschnitt (64F) vorgesehen, wobei eine mit der zweiten Datenschnittstelle (32B) verbundene Datenleitung (65F) zumindest abschnittsweise entlang des Ausgangsschlauchabschnitts (64F) geführt ist.

6. Infusionspumpe (10) nach einem der vorstehenden Ansprüche mit dem folgenden weiteren Merkmal:
a. die Leitungseinheit (50) weist zum Zwecke der Datenübertragung mindestens eine Anschlusseinrichtung (72) zum lösbaren Anschließen an eine Gegen-Anschlusseinrichtung (32) der Basiseinheit (20) auf,
vorzugsweise mit mindestens einem der folgenden Merkmale:
b. die Anschlusseinrichtung (72) und die Gegen-Anschlusseinrichtung (32) verfügen über korrespondierende Kontaktflächen zur galvanischen Kopplung, und/oder
c. es ist eine Mehrzahl von Anschlusseinrichtungen und Gegen-Anschlusseinrichtungen vorgesehen, und/oder
d. die Anschlusseinrichtung (72) ist am Tragteil (62) vorgesehen.

7. Infusionspumpe (10) nach einem der vorstehenden Ansprüche mit dem folgenden weiteren Merkmal:
a. die Infusionspumpe (10) weist mindestens eine mittels eines Ventilaktors (22A) elektrisch steuerbare Ventileinrichtung (22) auf, die dafür ausgebildet ist, den mindestens einen Fluideingang vom Fluidausgang zu trennen,
vorzugsweise mit mindestens einem der folgenden zusätzlichen Merkmale:
b. die Leitungseinheit (50) weist eine Mehrzahl von Fluideingängen auf, vorzugsweise fünf Fluideingänge, wobei insbesondere vorzugsweise jedem der Fluideingänge eine Ventileinrichtung (22) zugeordnet ist, und/oder
c. dem Fluidausgang ist eine Ventileinrichtung (22) zugeordnet, und/oder
d. der Ventilaktor (22A) ist Teil der Basiseinheit (20), und/oder
e. die mindestens eine Ventileinrichtung (22) ist als bistabile Ventileinrichtung (22) ausgebildet, so dass die Ventileinrichtung (22) ohne Stromzufuhr in einem Schließzustand und einem Öffnungszustand verbleiben kann, oder
f. die mindestens eine Ventileinrichtung (22) ist als monostabile Ventileinrichtung (22) ausgebildet und weist eine dem Ventilaktor (22A) entgegenwirkende Rückstellfeder (22B) auf, so dass die Ventileinrichtung (22) ohne Stromzufuhr einen Schließzustand einnimmt.

8. Infusionspumpe (10) nach einem der vorstehenden Ansprüche mit mindestens einem der folgenden weiteren Merkmale:
a. das Steuergerät (30) ist dafür ausgebildet, eine eindeutige Kennung über die ersten und/oder über die zweite Datenschnittstelle (32A, 32B) einzulesen, und/oder
b. das Steuergerät (30) ist dafür ausgebildet, über die erste Datenschnittstelle (32A) einen patientenspezifischen Therapieplan von einem angeschlossenen Medikamentenspeicher (110) einzulesen, und/oder
c. das Steuergerät (30) ist dafür ausgebildet, in Reaktion auf das Anschließen eines Medikamentenspeichers (110) und/oder eines Patienten (2) einen Therapieplan einzulesen und beim späteren Anschluss eines weiteren Medikamentenspeichers (110) zu prüfen, ob dieser mit dem Therapieplan in Einklang steht, und/oder
d. das Steuergerät (30) ist dafür ausgebildet, beim Anschluss eines Medikamentenspeichers (110) zu prüfen, ob der Medikamentenspeicher (110) am hierfür vorgesehenen Anschlussstück (66A-66E) angeschlossen worden ist, und/oder
e. das Steuergerät (30) ist dafür ausgebildet, die Echtheit eines angeschlossenen Medikamentenspeichers (110) zu prüfen, insbesondere durch Überprüfung einer über die erste Datenschnittstelle (32A) übermittelte digitale und vorzugsweise kryptographische Authentifizierung, und/oder
f. das Steuergerät (30) ist dafür ausgebildet, über die erste Datenschnittstelle (32A) die Art des Medikaments in einem Medikamentenspeicher (110) zu ermitteln und mit einem Therapieplan zu vergleichen, und/oder
g. das Steuergerät (30) ist dafür ausgebildet, über die erste Datenschnittstelle (32A) Daten von einem einem Medikamentenspeicher (110) zugeordneten Sensor (114) einzulesen und/oder über die zweite Datenschnittstelle (32B) Daten von einem dem Patienten (2) zugeordneten Sensor (124) einzulesen, und/oder
h. das Steuergerät (30) ist dafür ausgebildet, über die Tätigkeit der Infusionspumpe (10) ein Protokoll zu führen, wobei insbesondere eines oder mehrere der folgenden Ereignisse und/oder Daten protokolliert werden:
- Anschließen eines Patienten (2) an der Infusionspumpe (10), und/oder
- Anschließen eines Medikamentenspeichers (110) an der Infusionspumpe (10), vorzugsweise einschließlich Daten über die Art des Medikaments und/oder umfassend eine eindeutige Kennzeichnung der Charge, und/oder
- Eingangswerte von mindestens einem Sensor (114), der einem Medikamentenspeicher (110) zugeordnet ist, und/oder
- Eingangswerte von mindestens einem Sensor (124), der dem Patienten zugeordnet ist, und/oder
i. das Steuergerät (30) ist dafür ausgebildet, mit einem externen Netzwerk (212) verbunden zu werden und über dieses externe Netzwerk (212)
- einen patientenspezifischen Therapieplan und/oder Daten einer Patientenakte einzulesen, und/oder
- ein Protokoll über die Tätigkeit des Infusionspumpe (10) zu versenden, und/oder
j. das Steuergerät (30) ist dafür ausgebildet, die Aktivierung des Pumpaktors (24) und/oder das Öffnen einer Ventileinrichtung (22) zu unterbinden oder zu unterbrechen, wenn über die Datenschnittstellen (32A, 32B) eingelesene Daten eine von Sollparametern abweichende Situation repräsentieren, und/oder
k. das Steuergerät (30) ist dafür ausgebildet, ein Warnsignal zu generieren und insbesondere in visueller oder akustischer Form abzugeben, wenn über die Datenschnittstellen (32A, 32B) eingelesene Daten eine von Sollparametern abweichende Situation repräsentieren.

9. Infusionspumpe (10) nach einem der vorstehenden Ansprüche mit mindestens einem der folgenden weiteren Merkmale:
a. die Infusionspumpe (10) weist ein gemeinsames Pumpengehäuse auf, innerhalb dessen und/oder an dessen Vorderseite mindestens der mindestens eine Pumpaktor (24) sowie mindestens eine Ventileinrichtung (22) angeordnet ist, und/oder
b. mindestens im Bereich der Ventileinrichtung (22) bzw. im Bereich des mindestens eines Pumpaktors (24) ist die Fluidleitung (64) ohne Datenleitungen ausgebildet, und/oder
c. mindestens ein Anschlussstück (66A-66F) ist als Luer-Konnektor gemäß der Normenfamilie ISO 80369 ausgebildet, wobei vorzugsweise alle Anschlussstück (66A-66F) als Luer-Konnektoren gemäß der Normenfamilie ISO 80369 ausgebildet sind.

10. Infusionspumpe (10) nach einem der vorstehenden Ansprüche mit dem folgenden weiteren Merkmal:
a. die Infusionspumpe (10) weist eine Anzeigeeinrichtung (34) auf,
vorzugsweise mit mindestens einem der folgenden zusätzlichen Merkmale:
b. das Steuergerät (30) ist dafür ausgebildet, auf dieser Anzeigeeinrichtung (34) folgendes darzustellen:
- Eingangswerte von mindestens einem Sensor (114), der dem Medikamentenspeicher (110) zugeordnet ist, und/oder
- Eingangswerte von mindestens einem Sensor (124), der dem Patienten (2) zugeordnet ist, und/oder
- Informationen zu einer derzeit ablaufenden Infusionsbehandlung in für medizinisches Fachpersonal oder den Patienten geeigneter Form, und/oder
c. das Steuergerät (30) ist dafür ausgebildet, über die Anzeigeeinrichtung (34) eine Videokonferenz darzustellen.

11. Infusionspumpe (10) nach einem der vorstehenden Ansprüche mit dem folgenden weiteren Merkmal:
a. die Infusionspumpe (10) ist dafür ausgebildet, ein Feedback des Patienten entgegenzunehmen,
insbesondere mit dem folgenden zusätzlichen Merkmal:
b. die Infusionspumpe (10) weist Eingabemittel auf, insbesondere in Form einer als Touchscreen ausgebildeten Anzeigeeinrichtung (34) oder in Form hiervon getrennter Bedienelement, mittels derer der Patient Feedback geben kann, und/oder
c. die Infusionspumpe (10) ist dafür ausgebildet, eine laufende Infusionsbehandlung in Abhängigkeit des Feedbacks anzupassen oder zu unterbrechen.

12. Infusionsanordnung (100) mit den folgenden Merkmalen:
a. die Infusionsanordnung (100) weist eine Infusionspumpe (10) nach einem der vorstehenden Ansprüche auf, und
b. die Infusionsanordnung (100) weist mindestens einen mit einem flüssigen Medikament befüllten Medikamentenspeicher (110) auf, der an dem mindestens einen Fluideingang der Infusionspumpe (10) angeschlossen ist, und
c. die Infusionsanordnung (100) weist mindestens eine patientenseitige Zugangseinrichtung (120) zur Einbringung des flüssigen Medikaments in den Körper eines Patienten auf, und
d. das Steuergerät der Infusionspumpe (10) ist über die erste und die zweite Datenschnittstelle mitdem Medikamentenspeicher (110) und mit der Zugangseinrichtung (120) verbunden.

13. Infusionsanordnung (100) nach Anspruch 12 mit den folgenden Merkmalen:
a. der Medikamentenspeicher (110) ist mit einem elektronischen Speicher (112) versehen, der über die erste Datenschnittstelle (32A) durch das Steuergerät (30) auslesbar ist, und/oder
b. der Medikamentenspeicher (110) ist mit mindestens einem Sensor (114) versehen, der über die erste Datenschnittstelle (32A) durch das Steuergerät (30) auslesbar ist,
vorzugsweise mit dem folgenden zusätzlichen Merkmal:
c. der mindestens eine Sensor (114) ist ein Temperatursensor oder ein Sensor zur Erfassung von optischen Eigenschaften der Flüssigkeit oder ein Sensor zur Erfassung der Konzentration der Flüssigkeit.

14. Infusionsanordnung (100) nach Anspruch 12 oder Anspruch 13 mit den folgenden Merkmalen:
a. die Zugangseinrichtung (120) ist mit einem elektronischen Speicher (122) versehen, der über die zweite Datenschnittstelle (32B) durch das Steuergerät (30) auslesbar ist, und/oder
b. die Zugangseinrichtung (120) ist mit mindestens einem Sensor (124) versehen, der über die zweite Datenschnittstelle (32B) durch das Steuergerät (30) auslesbar ist,
vorzugsweise mit dem folgenden zusätzlichen Merkmal:
c. der mindestens eine Sensor (124) ist ein Temperatursensor oder ein Blutdrucksensor oder ein Sauerstoffsensor oder ein Sensor zur Messung von Herzströmen oder ein Sensor zur Messung von Gewebeimpedanz.

15. Infusionssystem (200) mit den folgenden Merkmalen:
a. das Infusionssystem (200) weist eine Infusionspumpe (10) nach einem der Ansprüche 1 bis 11 oder eine Infusionsanordnung (100) nach einem der Ansprüche 12 bis 14 auf, und
b. das Infusionssystem (200) weist mindestens einen Server (210) auf, der über ein Netzwerk (212) mit der Infusionspumpe (10) verbunden ist, und
c. das Steuergerät (30) der Infusionspumpe (10) ist dafür ausgebildet, Daten vom Server (210) abzurufen und/oder Daten auf dem Server (210) abzulegen.

16. Verfahren zum Betrieb einer Infusionspumpe (10) nach einem der Ansprüche 1 bis 11 mit den folgenden Merkmalen:
a. es werden Daten von dem Medikamentenspeicher (110) über die erste Datenschnittstelle (32A) gelesen, und
b. es werden Daten über den Patienten (2) über die zweite Datenschnittstelle (32A) gelesen, und
c. es erfolgt eine gemeinsame Datenverarbeitung der Daten, und
d. in Abhängigkeit des Ergebnisses der Datenverarbeitungwird die Flüssigkeitsförderung mittels der Infusionspumpe (10) gestartet oder nicht gestartet und/oder es wird mindestens eine Ventileinrichtung (22) der Infusionspumpe (10) geschlossen oder geöffnet,
vorzugsweise mit mindestens einem der folgenden weiteren Schritte:
e. im Zuge der Datenverarbeitung wird die Echtheit eines angeschlossenen Medikamentenspeichers (110) geprüft, und/oder
f. im Zuge der Datenverarbeitung wird geprüft, ob ein angeschlossener Medikamentenspeichers (110) am richtigen Anschlussstück (66A-66E) angeschlossen ist, und/oder
g. im Zuge der Datenverarbeitung wird ein Therapieplan eingelesen, wobei vorzugsweise
- das Einlesen des Therapieplans über die erste Datenschnittstelle (32A) oder über die zweite Datenschnittstelle (32B) erfolgt oder
- das Einlesen des Therapieplans erfolgt über ein externes Netzwerk (212), wobei hierfür ein eindeutiges Kennzeichen herangezogen wird, welches zuvor über die erste Datenschnittstelle (32A) oder über die zweite Datenschnittstelle (32B) eingelesen wurde.
